# EUROPÄISCHE PATENTSCHRIFT

(11) **EP 1 244 418 B1**
(45) Veröffentlichungstag und Bekanntmachung des Hinweises auf die Patenterteilung: **30.06.2004**
(21) Anmeldenummer: 00990764.3
(22) Anmeldetag: 15.12.2000
(51) Int. Cl.: A61K 7/00, A61K 7/48

(54) **KOSMETISCHE ODER DERMATOLOGISCHE ZUBEREITUNGEN VOM TYP ÖL-IN-WASSER**
OIL-IN-WATER TYPE COSMETIC OR DERMATOLOGICAL PREPARATIONS
PREPARATIONS COSMETIQUES OU DERMATOLOGIQUES DU TYPE HUILE DANS EAU

(30) Priorität: 05.01.2000 DE 10000210
(43) Veröffentlichungstag der Anmeldung: 02.10.2002
(73) Patentinhaber: Beiersdorf AG, 20253 Hamburg (DE)
(72) Erfinder: MÜLLER, Anja, 23843 Rümpel (DE)
(86) Internationale Anmeldenummer: PCT/EP2000/012780
(87) Internationale Veröffentlichungsnummer: WO 2001/049252

(56) Entgegenhaltungen:
- EP-A- 0 821 944
- EP-A- 1 077 059
- DE-A- 3 304 896
- DE-A- 4 337 041
- DE-A- 19 725 087

## Beschreibung

Die vorliegende Erfindung betrifft kosmetische und dermatologische Zubereitungen vom Typ ÖI-in-Wasser, insbesondere fließfähige O/W-Emulsionen, ganz besonders sprühbare O/W-Emulsionen, weiche eine Viskosität von weniger als 2000 mPa·s haben, sowie ein Verfahren zur Stabilisierung von O/W-Formulierungen mittels Dichteangleich der Phasen.

Kosmetische Zubereitungen werden im wesentlichen zur Pflege der Haut benutzt. Die menschliche Haut übt als größtes Organ des Menschen zahlreiche lebenswichtige Funktionen aus. Mit durchschnittlich etwa 2 m² Oberfläche beim Erwachsenen kommt ihr eine herausragende Rolle als Schutz- und Sinnesorgan zu. Unter ihren vielen Funktionen (beispielsweise zur Wärmeregulation) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe (z. B. Schmutz, Chemikalien, Mikroorganismen). Außerdem kommt ihr eine bedeutende Rolle als Regulations- und Zielorgan im menschlichen Stoffwechsel zu.

Die kosmetische Hautpflege dient in erster Linie dazu, die natürliche Funktion der Haut als Barriere gegen Umwelteinflüsse und gegen den Verlust von körpereigenen Stoffen (neben Wasser auch natürliche Fette, Elektrolyte etc.) zu stärken oder wiederherzustellen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. UV-A-Strahlung (320 bis 400 nm) ist im Hinblick auf die Auslösung photodynamischer, speziell phototoxischer Reaktionen und chronischer Veränderungen der Haut noch weitaus gefährlicher als UV-B-Strahlung. So reicht selbst die UV-A-Strahlung unter ganz normalen Alltagsbedingungen aus, um innerhalb kurzer Zeit die Kollagen- und Elastinfasem zu schädigen. Auch kann der schädigende Einfluß der UV-B-Strahlung durch UV-A-Strahlung noch verstärkt werden.

Ferner können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können kosmetischen bzw. dermatologischen Formulierungen neben UV-Filtersubstanzen zusätzlich Antioxidantien und/oder Radikalfänger zugesetzt werden.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

Den bei weitem wichtigsten Produkttyp im Bereich der Hautpflegemittel stellen Emulsionen dar. Emulsionen sind disperse Zwei- oder Mehrphasensysteme, wobei kosmetische Emulsionen aus mindestens einer Fettphase (Fette und mineralische Öle, Fettsäureester, Fettalkohole etc.) und mindestens einer Wasserphase (Wasser, Glycerin, Glykole usw.) bestehen, die mit Hilfe von Emulgatoren in Form feinster Tröpfchen ineinander verteilt werden. Liegt die Ölphase fein verteilt in der Wasserphase vor, so handelt es sich um eine Öl-in-Wasser-Emulsion (O/W-Emulsion, z. B. Milch). Der Grundcharakter einer O/W-Emulsion ist durch das Wasser geprägt, d. h. sie wirkt weniger fettend auf der Haut, ist eher mattierend und zieht schneller in die Haut ein als eine W/O-Emulsion.

Obwohl Emulsionen vom thermodynamischen Standpunkt aus betrachtet instabile Systeme sind, gelingt es, kosmetische Emulsionen von jahrelanger Stabilität herzustellen. Eine Emulsion wird als stabil bezeichnet, wenn sich über einen vorgegebenen Zeitraum hinweg keine meßbaren zeitlichen und örtlichen Änderungen der Tropfengrößenverteilung feststellen lassen.

Die Stabilität bzw. Instabilität von Emulsionen hängt von verschiedenen Faktoren ab. Zum einen neigt beispielsweise die Wasserphase einer W/O-Emulsion, da Wasser- und Ölphase unterschiedliche Dichten haben, zur Sedimentation. Die Ölphase einer O/W-Emulsion hat dementsprechend eine Tendenz zum Aufrahmen.

Ferner kann es aufgrund der Anziehungskräfte zwischen den feinverteilten Tröpfchen der dispersen Phase zu einer Tropfenaggregation kommen, wobei die einzelnen Tröpfchen eines Aggregates zunächst durch einen dünnen Film kontinuierlicher Phase voneinander getrennt bleiben. Die ursprüngliche Tropfengrößenverteilung ändert sich dabei nur scheinbar und kann in diesem Fall durch Rühren oder Schütteln wiederhergestellt werden.

Allerdings können die sich berührenden Tröpfchen darüber hinaus auch zusammenfließen, was zu einer echten Änderung der Tropfengrößenverteilung führt, die nur durch Energiezufuhr wieder verändert werden kann. Dieser Vorgang wird als Koaleszenz bezeichnet. Der Prozeß der Koaleszenz läuft um so langsamer ab, je viskoser die äußere Phase der Emulsion ist.

Die beschriebenen Vorgänge können einzeln oder zusammen ablaufen. Oft initiiert oder verstärkt ein Vorgang den anderen. So wird z. B. durch die Bildung von Aggregaten in O/W-Emulsionen das Aufrahmen der Ölphase beschleunigt. Geht der disperse Zustand einer Emulsion teilweise oder auch ganz verloren, so trennen sich die beiden Phasen, und man spricht vom Brechen der Emulsion.

Zur Stabilisierung von Emulsionen über einen längeren Zeitraum hinweg werden dementsprechend Hilfsmittel benötigt, die das Entmischen der beiden Phasen unterbinden, mindestens aber so lange verzögern, bis die Emulsion ihre Bestimmung erfüllt hat.

Diese Hilfsmittel sollen zum einen die Grenzfläche stabilisieren, indem sie verhindern, daß die Tröpfchen der dispersen Phase zusammenfließen. Im Idealfall bewirken diese Stoffe darüber hinaus eine Abstoßung der Tröpfchen, welche verhindert, daß sich diese annähern, so daß eine Zusammenballung (Aggregatbildung) vermieden werden kann.

Zum anderen werden Hilfsstoffe dazu eingesetzt, dem Aufrahmen bzw. Sedimentieren der Phasen entgegenzuwirken.

Emulgatoren sind grenzflächenaktive Substanzen, die in der Lage sind, die Grenzflächenspannung zwischen Öl- und Wasserphase zu vermindern, indem sie sich bevorzugt an der Grenzfläche zwischen diesen beiden anlagern. Dies wird durch ihren amphipilen Molekülaufbau ermöglicht: Emulgatoren besitzen wenigstens eine polare (hydrophile) Gruppe und wenigstens eine unpolare (lipophile) Gruppe. Damit sind sie sowohl in der hydrophilen als auch in der lipophilen Phase löslich. Der in der entsprechenden Phase besser lösliche Teil ragt in diese hinein und senkt dadurch die Grenzflächenspannung zwischen beiden Phasen.

Der Versuch der Klassifizierung von Emulgatoren ist schwierig, da diese zu chemisch sehr unterschiedlichen Kategorien gehören. Ein Emulgator ist um so effektiver, je schneller er die Grenzflächenspannung erniedrigt und je niedriger der Gleichgewichtswert der Grenzflächenspannung ist.

Darüber hinaus stabilisieren Emulgatoren Emulsionen auch dadurch, daß sie Grenzflächenfilme und damit sozusagen "physikalische" Barrieren ausbilden, wodurch die Aggregatbildung und die Koaleszenz der emulgierten Teilchen verhindert wird. Durch die Anlagerung des Emulgators an der Grenzfläche werden die Tröpfchen entweder aufgeladen, so daß sie sich gegenseitig abstoßen, oder es wird eine stabile, vielfach hochviskose oder sogar feste Schutzschicht um die Tröpfchen gebildet.

Für die praktische Herstellung kosmetischer oder dermatologischer Emulsionen reicht allerdings in der Regel der Einsatz eines oder mehrerer Emulgatoren allein nicht aus. Wesentliche Faktoren für die Stabilität kosmetischer oder dermatologischer Zubereitungen sind ferner:
. feinste Verteilung der beiden Phasen ineinander:
   Je kleiner die dispergierten Teilchen sind, um so stabiler ist die Emulsion.
. hohe Viskosität der äußeren Phase
. ein stabiler Grenzflächenfilm
. ein ausgewogenes Phasenvolumenverhältnis

Das Emulgatorsystem muß daher meist zusätzlich zum eigentlichen Emulgator noch eine weitere Komponente enthalten, die als Co-Emulgator, Stabilisator oder je nach Wirkmechanismus auch als Konsistenzgeber, Verdickungsmittel oder Schutzkolloid usw. bezeichnet wird.

Durch diese Stoffe, im folgenden der Einfachheit halber Stabilisatoren genannt, wird die Stabilität einer Emulsion erhöht. Stabilisatoren müssen nicht grenzflächenaktiv, können aber amphiphil aufgebaute Verbindungen sein.

Eine Möglichkeit, Emulsionen zu stabilisieren, ist nach dem oben gesagten, die Viskosität der äußeren Phase zu erhöhen. Diese Viskositätserhöhung bewirkt in der Regel eine erhebliche Verminderung der Beweglichkeit der dispergierten Tröpfchen, wodurch die Sedimentations- bzw. Aufrahmgeschwindigkeit vermindert wird. Damit einhergehend treffen die Tröpfchen ferner weniger häufig aufeinander, was eine geringere Koaleszenzneigung zur Folge hat.

Die Viskosität der äußeren Phase läßt sich beispielsweise durch Zugabe von Verdikkungsmitteln erhöhen, welche z. B. Gele und/oder lamellare Flüssigkristalle ausbilden. Auch Emulgatoren sind im Prinzip in der Lage, durch die Bildung von Emulgatorgelnetzwerken die Viskosität einer Flüssigkeit zu erhöhen. Allerdings wird hierfür eine vergleichsweise hohe Menge an Emulgator benötigt, da Gelnetzwerke erst dann gebildet werden, wenn die gesamte Grenzfläche zwischen den Phasen mit Emulgatormolekülen belegt ist.

Das Brechen einer Emulsion läßt sich ferner durch Wahl eines geeigneten Phasenvolumenverhältnisses verhindern. Zur Verdeutlichung dieser Tatsache stelle man sich eine Emulsion als System aus Metallkugeln gleichen Durchmessers (innere Phase) und einer Flüssigkeit (äußere Phase) vor. Eine Sedimentation bzw. ein Aufrahmen kann - in diesem einfachen Modell - dann nicht mehr stattfinden, wenn die gesamte Flüssigkeit mit Metallkugeln ausgefüllt ist. Dies ist - nimmt man eine dichteste Kugelpackung als Verteilung an - gerade bei einem Verhältnis von 1 : 2 der Fall, d. h. wenn die Emulsion zu 2/3 aus innerer Phase besteht. Es ist offensichtlich, daß die Viskosität einer Emulsion bei wachsendem Anteil an innerer Phase zunimmt, da hierdurch die Beweglichkeit der dispergierten Tröpfchen eingeschränkt wird.

Natürlich ist dem Fachmann eine Vielzahl von Möglichkeiten bekannt, stabile O/W-Zubereitungen zur kosmetischen oder dermatologischen Anwendung zu formulieren, beispieisweise in Form von Cremes und Saiben, die im Bereich von Raum- bis Hauttemperatur streichfähig sind, oder als Lotionen und Milche, die in diesem Temperaturbereich eher fließfähig sind. Dabei kommt es neben der Wahl des "richtigen" Emulgators bzw. Emulgatorsystems insbesondere auch auf die weitere Zusammensetzung der Zubereitung an.

O/W-Emulsionen werden in der Regel durch Verdickungsmittel, welche die Viskosität der wäßrigen Phase erhöhen, stabilisiert. Hierzu eignen sich beispielsweise Polyacrylate (Carbomer) und weitere organische Verdickungsmittel. Ein Nachteil dieser Methode der Stabilitätsverbesserung ist die Empfindlichkeit dieser Formulierungen gegen Elektrolyte. Ferner sind auf diese Weise naturgemäß höherviskose Formulierungen (wie Cremes oder Salben) herzustellen. Auch die Stabilisierung von O/W-Emulsionen über das Phasenvolumenverhältnis führt nach dem gesagten zu zähflüssigen Formulierungen.

Emulsionen von "flüssiger" (= fließfähiger) Konsistenz finden in der Kosmetik beispielsweise als Pflege-, Reinigungs-, Gesichts- oder Handlotion Verwendung. Sie haben in der Regel eine Viskosität von etwa 2000 mPa·s bis zu etwa 10 000 mPa·s. Der Stabilität von fließfähigen Emulsionen ist besondere Aufmerksamkeit zu widmen, da die erheblich größere Beweglichkeit der Teilchen eine schnellere Koaleszenz fördert.

Auch diese flüssigen Emulsionen des Standes der Technik sind - da auch sie i. a. Verdickungsmittel enthalten - gegenüber höheren Elektrolytkonzentrationen nicht stabil, was sich in einer Phasentrennung äußern kann. Es ist aber häufig wünschenswert, bestimmte Elektrolyte, wie beispielsweise wasserlösliche UV-Filter, einzusetzen, um deren sonstige physikalische, chemische bzw. physiologische Eigenschaften nutzen zu können. Zwar läßt sich in vielen Fällen durch geeignete Wahl des Emulgatorsystems in gewissem Maß Abhilfe schaffen, es treten dann aber ebensooft andere Nachteile auf.

Die angesprochenen Nachteile können beispielsweise darin liegen, daß vergleichsweise große Mengen an einem oder mehreren Emulgatoren erforderlich sind (z. B. 3 Gew.-% oder mehr). Da aber auch Emulgatoren - wie letztendlich jede chemische Substanz - im Einzelfall allergische oder auf Überempfindlichkeit des Anwenders beruhende Reaktionen hervorrufen können (obwohl die Verwendung der üblichen kosmetischen Emulgatoren i. a. natürlich völlig unbedenklich ist) ist es wünschenswert, den Emulgatorgehalt einer kosmetischen oder dermatologischen Formulierung so klein wie möglich zu halten.

Emulsionen mit einer sehr geringen Viskosität (dünnflüssige bzw. sprühbare Emulsionen) sind nach dem oben gesagten bislang - wenn überhaupt - nur sehr aufwendig zu formulieren. Dementsprechend ist das Angebot an derartigen Formulierungen äußerst gering. Gleichwohl könnten solche Formulierungen dem Verbraucher bisher nicht gekannte kosmetische Leistungen bieten.

Überhaupt haben dünnflüssige Zubereitungen des Standes der Technik häufig den Nachteil, daß sie auf einen engen Anwendungsbereich oder eine eingeschränkte Rohstoffauswahl begrenzt sind.

Eine Aufgabe der vorliegenden Erfindung war es, den Stand der Technik um OIW-Zubreitungen zur kosmetischen oder dermatologischen Anwendung zu bereichern.

Ferner war Aufgabe der Erfindung, Zubereitungen vom Typ Öl-in-Wasser herzustellen, welche eine geringe bzw. sehr geringe Viskosität haben und nicht die Nachteile des Standes der Technik aufweisen. Eine weitere Aufgabe der Erfindung war, Lösungswege zu kosmetischen oder dermatologischen, möglichst dünnflüssigen O/W-Emulsionen aufzudecken, welche gegenüber erhöhten Elektrolytkonzentrationen stabil sind.

Weiterhin war es Aufgabe der vorliegenden Erfindung, ein Verfahren zur Stabilisierung von O/W-Formulierungen zu finden.

Erstaunlicherweise werden diese Aufgaben gelöst durch kosmetische oder dermatologische Zubereitungen vom Typ ÖI-in-Wasser, welche eine Ölphase, in welche ein oder mehrere Citronensäureester ausgewählt aus der Gruppe bestehend aus Acetyltriethylcitrat, Tributylcitrat und Acetyltributylcitrat eingearbeitet sind, und eine Wasserphase enthalten, wobei die Dichtedifferenz zwischen der Ölphase und der Wasserphase (bestimmbar mit einem rechnenden digitalen Dichtemesser vom Typ DMA 45 der Firma chempro/PA bei 25 °C) nicht größer als 0,01 g·cm⁻³ ist sowie gewünschtenfalls übliche kosmetische oder dermatologische Hilfs-, Zusatzund/oder Wirkstoffe enthaltend.

Gegenstand der Erfindung ist ferner ein Verfahren zur Stabilisierung von O/W-Formulierungen, dadurch gekennzeichnet, daß die Dichte der Ölphase durch Zugabe von einem oder mehreren Citronensäureestem der allgemeinen Strukturformel 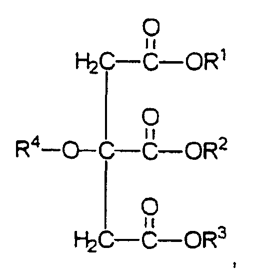 worin R¹, R² und R³ unabhängig voneinander aus der Gruppe Ethyl, Propyl und Butyl gewählt werden und R⁴ ein Wasserstoffatom oder eine CH₃(CO)-Gruppe darstellt, der Dichte der Wasserphase derart angeglichen wird, daß die Dichtedifferenz der beiden Phasen nicht größer als 0,01 g·cm⁻³ ist.

Auch nach diesem Verfahren erhältliche O/W-Formulierungen sind Gegenstand der vorliegenden Erfindung.

Vorteilhafte Citronensäureester im Sinne der vorliegenden Erfindung sind solche, für die R¹, R² und R³ identische Reste darstellen. Insbesondere vorteilhaft sind Triethylcitrat, Acetyltriethylcitrat, Tributylcitrat und Acetyltributylcitrat. Erfindungsgemäß bevorzugt sind ferner Citronensäureester, welche in Wasser nur schwer oder gar nicht löslich sind, d. h. insbesondere solche, für die mindestens einer der Reste R¹, R² und R³ einen Butylrest darstellt und/oder für die R⁴ ein Acetylrest ist.

Die erfindungsgemäßen Zubereitungen stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die erfindungsgemäßen Zubereitungen haben eine ausgezeichnete Stabilität gegenüber einem Zerfall in Öl- und Wasserphasen und zeigen sehr gute sensorische Eigenschaften, wie beispielsweise die Verteilbarkeit auf der Haut oder das Einzugsvermögen in die Haut.

Es war insbesondere überraschend, daß die erfindungsgemäßen Zubereitungen auch ohne Zusatz weiterer Stabilisatoren - wie beispielsweise Konsistenzgeber, Verdickungsmittel oder Schutzkolloide usw. - außerordentlich stabil sind. Dies war insbesondere für fließfähige oder gar sprühbare Formulierungen in keiner Weise vorhersehbar.

Die erfindungsgemäßen Zubereitungen stellen in jeder Hinsicht eine Bereicherung des Standes der Technik in bezug auf O/W-Emulsionen, insbesondere in bezug auf fließfähige oder sprühbare O/W-Emulsionen dar.

Ferner sind nach dem erfindungsgemäßen Verfahren in erstaunlich einfacher Weise sehr stabile O/W-Formulierungen, beispielsweise sprühbare Formulierungen mit einem hohen Lichtschutzfaktor erhältlich.

Es ist erfindungsgemäß besonders vorteilhaft, wenn die Zubereitungen deutlich weniger als 1 Gew.-% (bezogen auf das Gesamtgewicht der Zubereitungen) eines oder mehrerer Emulgatoren enthalten.

Es ist ferner vorteilhaft, wenn der mittlere Durchmesser der Öltröpfchen der erfindungsgemäßen Formulierungen kleiner als 50 µm ist. Bevorzugt im Sinne der vorliegenden Erfindung sind außerdem Formulierungen, deren Gesamtdichte größer ist als 0,9 g·cm⁻³, insbesondere größer als 0,95 g·cm⁻³.

Es kann femer vorteilhaft sein, wenn die erfindungsgemäßen O/W-Formulierungen, obwohl es nicht notwendig ist, auch Stabilisatoren enthalten, welche vorteilhaft aus der Gruppe der Verdickungsmittel gewählt werden. Es ist vorteilhaft, den Gehalt an dem oder den Verdickungsmitteln aus dem Bereich 0,05 Gew.-% bis 0,15 Gew.-% zu wählen, bezogen auf das Gesamtgewicht der Zubereitungen.

Die Ölphase der erfindungsgemäßen O/W-Formulierungen weist vorteilhaft einen hohen Gehalt an erfindungsgemäßen Citratestem auf oder kann sogar vollständig aus solchen Estern bestehen. Es kann darüber hinaus aber auch vorteilhaft sein, weitere Öle zu verwenden, beispielsweise um ein besseres Hautgefühl der Formulierungen zu erreichen oder die Pflegeleistung der Produkte zu erhöhen. Die erfindungsgemäßen kosmetischen und dermatologischen Zubereitungen können dementsprechend ferner Ölkomponenten enthalten, wie sie üblicherweise in solchen Zubereitungen verwendet werden.

Kosmetische oder dermatologische Hilfs- und Zusatzstoffe

Sollen die O/W-Formulierungen gemäß der vorliegenden Erfindung Emulgatoren enthalten, so sind solche Emulgatoren vorteilhaft zu verwenden, welche zur Herstellung von O/W-Emulsionen geeignet sind, wobei diese sowohl einzeln als auch in beliebigen Kombinationen miteinander vorliegen können.

Vorteilhaft werden der oder die Emulgatoren aus der Gruppe gewählt, die die folgenden Verbindungen umfaßt:
Polyglyceryl-2-Dipolyhydroxystearat, PEG-30-Dipolyhydroxystearat, Cetyldimethiconcopolyol, Glykoldistearat, Glykoldilaurat, Diethylenglykoldilaurat, Sorbitantrioleat, Glykololeat, Glyceryldilaurat, Sorbitantristearat, Propylenglykolstearat, Propylenglykollaurat, Propylenglykoldistearat, Sucrosedistearat, PEG-3 Castor Oil, Pentaerythritylmonostearat, Pentaerythritylsesquioleat, Glyceryloleat, Glycerylstearat, Glyceryldiisostearat, Pentaerythritylmonooleat, Sorbitansesquioleat, Isostearyldiglycerylsuccinat, Glycerylcaprat, Palm Glycerides, Cholesterol, Lanolin, Glyceryloleat (mit 40 % Monoester), Polyglyceryl-2-Sesquiisostearat, Polyglyceryl-2-Sesquioleat, PEG-20 Sorbitan Beeswax, Sorbitanoleat, Sorbitanisostearat, Trioleylphosphat, Glyceryl Stearate und Ceteareth-20 (Teginacid von Th. Goldschmidt), Sorbitanstearat, PEG-7 Hydrogenated Castor Oil, Steareth-2, Oleth-2, Cetyl Alcohol und Ceteareth-30 (Emulgator E 2209 von Th. Goldschmidt), PEG-5-Soyasterol, PEG-6 Sorbitan Beeswax, Ceteth-2, Glycerylstearat SE, Methylglucosesesquistearate, PEG-10 Hydrogenated Castor Oil, Oleth-3, Sorbitanpalmitat, PEG-22/Dodecylglykol Copolymer, Polyglyceryl-2-PEG-4-Stearat, Laneth-5, Ceteth-3, Laureth-3, Stearyl Alcohol und Stearath-7 und Steareth- 10 (Emulgator E-2155 von Th. Goldschmidt), Oleth-5, Sorbitanlaurat, Laureth-4, PEG-4-Laurat, Polysorbat 61, Polysorbat 81, Beheneth-10, Polysorbat 65, Polysorbat 80, Laneth-10, Triceteareth-4-Phosphat, Triceteareth-4 Phosphate und Sodium C₁₄₋₁₇ Alkyl Sec Sulfonat (Hostacerin CG von Hoechst), PEG-8 Stearat, Glycerylstearat und PEG-100 Stearate (Arlacel 165 von ICI), Polysorbat 85, Trilaureth-4-Phosphat, PEG-25-Glyceryltrioleat, Oleth-10, Steareth-10, Ceteth-10, PEG-35 Castor Oil, Sucrosestearat, PEG-8-Oleat, Trioleth-8-Phosphat, PEG-40-Sorbitanlanolat, PEG-15-Glycerylricinoleat, Choleth-24 und Ceteth-24 (Solulan C-24 von Amerchol), C₁₂₋₁₅ Pareth-12, PEG-20-Glycerylisostearat, PEG-40 Hydrogenated Castor Oil, PEG-16 Soya Sterol, PEG-20-Glyceryloleat, PEG-20-Stearat, Polysorbat 80, PEG-20-Methylglucosesesquistearat, PEG-30-Glycerylisostearat, PEG-20-Glyceryllaurat, Ceteth-20, Ceteareth-25, PEG-30-Stearat, PEG-30-Glycerylstearat, Polysorbat 20, Laureth-23, PEG-40-Stearat, PEG-30-Glyceryllaurat, PEG-50-Stearat, PEG-100-Stearat, PEG-150-Laurat, Polyglyceryl-3-methylglucose Distearat, Ceteareth-12, Ceteareth-20 und Steareth-21, Ceteareth-6, PEG-40 Castor Oil, Natriumcetearylsulfat, Lecithin, Laureth-4-Phosphat, Propylenglykolstearat SE, PEG-25 Hydrogenated Castor Oil, PEG-54 Hydrogenated Castor Oil, Glycerylstearat SE, PEG-6 Caprylic/Capric Glycerides, Glyceryloleat und Propylenglykol, PEG-9-Stearat, Glyceryllanolat, Ceteth-2, Polysorbat 60, Glyceryfmyristat, Glycerylisostearat und Polyglyceryl-3 Oleat, Glyceryllaurat, PEG-40-Sorbitanperoleat, Laureth-4, Glycerinmonostearat, Ceteareth-3, Lanolinsäure, Isostearylglycerylether, Cetearyl Alcohol und Natriumcetearylsulfat, Steareth-2, PEG-22-Dodecylglykolcopolymer, Polyglyceryl-2-PEG-4-Stearat, Pentaerythrithylisostearat, Polyglyceryl-3-Diisostearat, Sorbitanoleat und Hydrogenated Castor Oil und Cera alba und Stearinsäure, Natriumdihydroxycetylphosphat und Isopropylhydroxycetylether, Methylglucosesesquistearat, Steareth-2 und PEG-8-Distearat, Steareth-20, Isosteareth-20, Methylglucosedioleat, Sorbitanoleat und PEG-2 Hydrogenated Castor Oil und Ozokerit und Hydrogenated Castor Oil, PEG-2 Hydrogenated Castor Oil, PEG-45-/Dodecylglykolcopolymer, Methoxy PEG-22-/Dodecylglykolcopolymer, Hydrogenated Coco Glycerides, Polyglyceryl-4-lsostearat, PEG-40-Sorbitanperoleat, PEG-40-Sorbitanperisostearat, PEG-20-Glycerylstearat, PEG-8-Beeswax, Laurylmethiconcopolyol, Polyglyceryl-2-Laurat, Stearamidopropyl-PG-dimoniumchloridphosphat, PEG-7 Hydrogenated Castor Oil, Triethylcitrat, PEG-20 Methylglucosesesquistearat, Glycerylstearatcitrat, Cetylphosphat, Polyglycerolmethylglucosedistearat, Poloxamer 101, Kaliumcetylphosphat, lsosteareth-10, Oleth-20, lsoceteth-20, Glycerylisostearat, Polyglyceryl-3-Diisostearate, Cetearylalkohol und PEG-20-Stearat.

Der oder die Emulgatoren werden insbesondere vorzugsweise gewählt aus der Gruppe der Fettsäuren, welche ganz oder teilweise mit üblichen Alkalien (wie z. B. Natrium- und Kaliumhydroxid, Natrium- und Kaliumcarbonat sowie Mono- und Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Die Emulgatoren werden erfindungsgemäß ferner vorzugsweise aus der Gruppe der gesättigten und/oder ungesättigten, verzweigten und/oder unverzweigten Fettalkohole mit 10 bis 40 Kohlenstoffatomen gewählt, besonders bevorzugt sind Butyloctanol, Butyldecanol, Hexyloctanol, Hexyldecanol, Octyldodecanol, Behenylalkohol (C₂₂H₄₅OH), Cetearylalkohol [eine Mischung aus Cetylalkohol (C₁₆H₃₃OH) und Stearylalkohol (C₁₈H₃₇OH)], Cetylarachidol [2-Hexadecyl-1-Eicosanol (C₃₆H₇₃OH),]. Lanolinalkohole (Wollwachsalkohole, die die unverseifbare Alkoholfraktion des Wollwachses darstellen, die nach der Verseifung von Wollwachs erhalten wird) und/oder 2-Tetradecyloctadecanol (C₃₂H₆₅OH). Vorteilhafte Ausführungsformen der beiden letztgenannten Fettalkohole sind unter den Handelsnamen Isofol 36 und Isofol 32 bei Condea erhältlich.

Die Liste der genannten Emulgatoren, die im Sinne der vorliegenden Erfindung eingesetzt werden können, soll selbstverständlich nicht limitierend sein.

Die erfindungsgemäßen Zubereitungen können vorteilhaft auch ein oder mehrere Hydrokolloide enthalten.

Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken.

Die Gruppe der Hydrokolloide läßt sich wie folgt einteilen in:
- organische, natürliche Verbindungen, wie beispielsweise Agar-Agar, Carrageen, Tragant, Gummi arabicum, Alginate, Pektine, Polyosen, Guar-Mehl, Johannisbrotbaumkemmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide,
- anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus 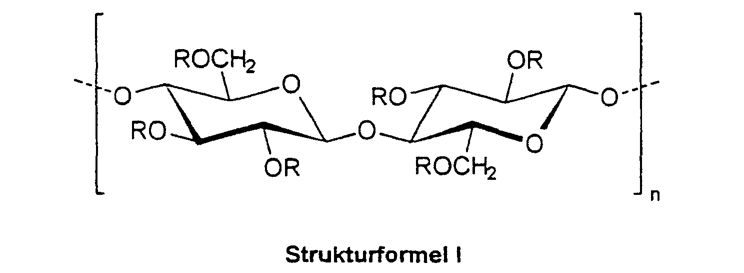 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das NatriumSalz des Glykolsäureethers der Cellulose, für welches R in Strukturformel I ein Wasserstoff und/oder CH₂-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, weiches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2×10⁶ bis 24×10⁶ produziert. Xanthan wird aus einer Kette mit β-1,4-gebundener Glucose (Cellulose) mit Seitenketten gebildet. Die Struktur der Untergruppen ("repeated units") besteht aus Glucose, Mannose, Glucuronsäure, Acetat und Pyruvat.

Erfindungsgemäß vorteilhafte Hydrokolloide sind ferner Polymere der Acrylsäure, insbesondere solche, die aus der Gruppe der sogenannten Carbomere oder Carbopole (Carbopol® ist eigentlich eine eingetragene Marke der B. F. Goodrich Company) gewählt werden. Carbopole sind Verbindungen der allgemeinen Strukturformel 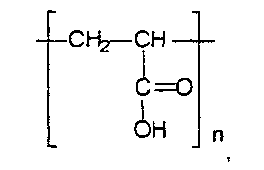 deren Molgewicht zwischen ca. 400 000 und mehr als 4 000 000 betragen kann. In die Gruppe der Carbopole gehören ferner Acrylat-Alkylacrylat-Copolymere, beispielsweise solche, die sich durch die folgende Struktur auszeichnen:

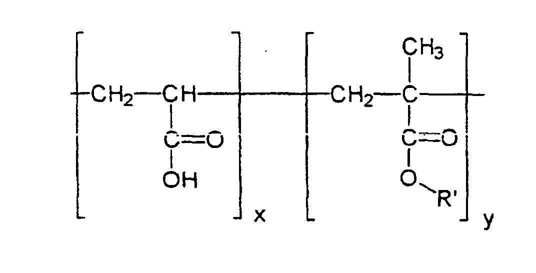

Darin stellen R' einen langkettigen Alkylrest und x und y Zahlen dar, welche den jeweiligen stöchiometrischen Anteil der jeweiligen Comonomere symbolisieren. Auch diese Carbopole sind vorteilhaft im Sinne der vorliegenden Erfindung.

Vorteilhafte Carbopole sind beispielsweise die Typen 907, 910, 934, 940, 941, 951, 954, 980, 981, 1342, 1382, 2984 und 5984, wobei diese Verbindungen einzeln oder in beliebigen Kombinationen untereinander vorliegen können. Besonders bevorzugt sind Carbopol 981, 1382 und 5984 (sowohl einzeln als auch in Kombination mit weiteren Hydrokolloiden).

Ferner vorteilhaft im Sinne der vorliegenden Erfindung sind die den Acrylat-Alkylacrylat-Copolymeren vergleichbaren Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer".

Insbesondere vorteilhaft sind die unter den Handelsbezeichnungen Pemulen TR1 und Pemulen TR2 bei der B. F. Goodrich Company erhältlichen.

Die Gesamtmenge an einem oder mehreren Hydrokolloiden wird in den fertigen kosmetischen oder dermatologischen Zubereitungen vorteilhaft kleiner als 1,0 Gew.-%, bevorzugt zwischen 0,01 und 0,5 Gew.-%, bezogen auf das Gesamtgewicht der Zubereitungen gewählt.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyloder -monobutylether, Propylenglykolmonomethyl, -monoethyl- oder -monobutylether, Diethylenglykolmonomethyl- oder -monoethylether und analoge Produkte, femer Alkohole niedriger C-Zahl, z.B. Ethanol, Isopropanol, 1,2-Propandiol und Glycerin.

Besonders vorteilhafte Zubereitungen werden femer erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, y -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄ Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Füllstoffe, Konservierungsmittel, Parfüme, Substanzen zum Verhindern des Schäumens, Farbstoffe, weitere oberflächenaktive Substanzen, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Mutatis mutandis gelten entsprechende Anforderungen an die Formulierung medizinischer Zubereitungen.

Die erfindungsgemäßen O/W-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die dünnflüssigen kosmetischen oder dermatologischen Mittel gemäß der Erfindung können beispielsweise als aus Aerosolbehältem, Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Als Treibmittel für aus Aerosolbehältem versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese zusätzlich mindestens eine UV-A-Filtersubstanz und/oder mindestens eine UV-B-Filtersubstanz.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure: 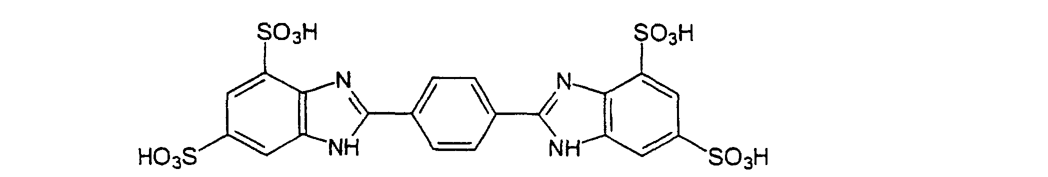 und ihre Salze, besonders die entsprechenden Natrium-, Kalium- oder Triethanolammonium-Salze, insbesondere das Phenylen-1,4-bis-(2-benzimidazyl)-3,3'-5.5'tetrasulfonsäure-bis-natriumsalz: 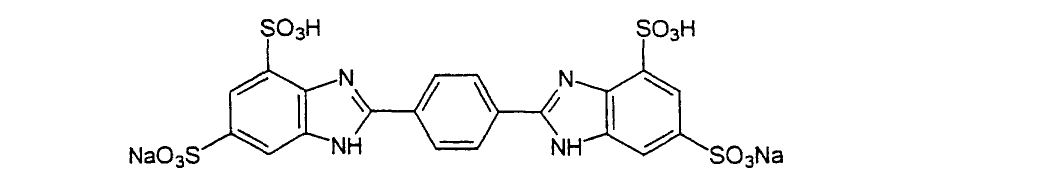 sowie das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-sulfonsäure) bezeichnet wird und sich durch die folgende Struktur auszeichnet:

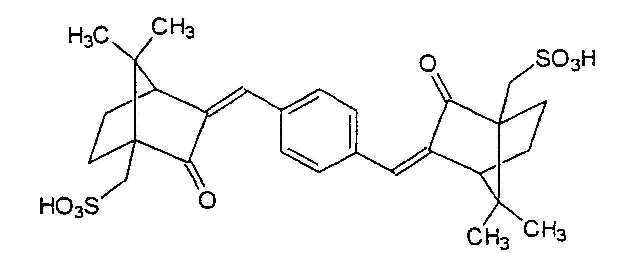

Vorteilhafte UV-Filtersubstanzen im Sinne der vorliegenden Erfindung sind femer sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 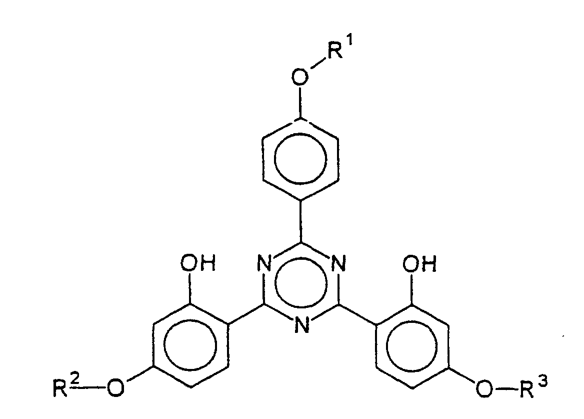 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch andere UV-Filtersubstanzen beschrieben, welche das Strukturmotiv 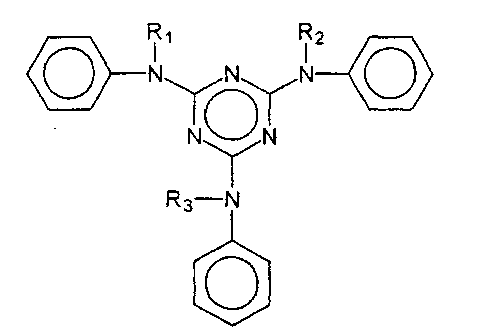 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 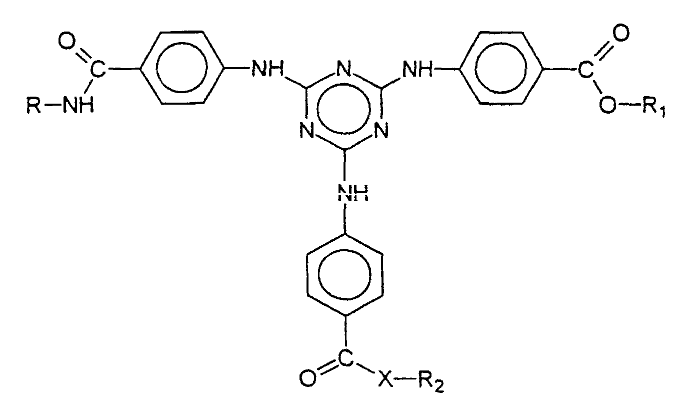 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 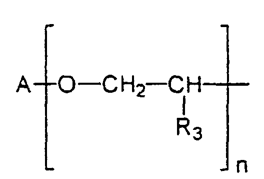 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine
Gruppe der Formel 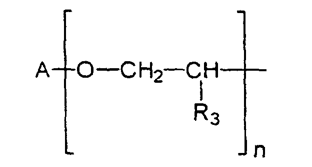 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 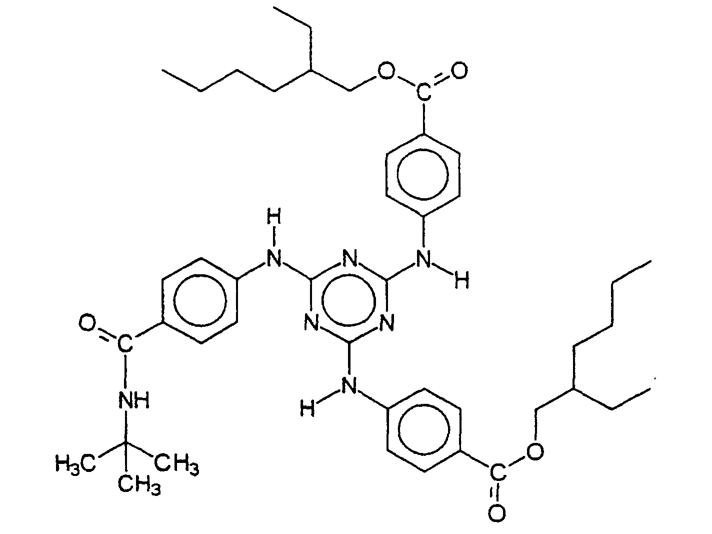 wiedergegeben wird und welche im Folgenden auch als Dioctylbutylamidotriazon bezeichnet wird.

Auch in der Europäischen Offenlegungsschrift 775 698 werden bevorzugt einzusetzende Bis-Resorcinyltriazinderivate beschrieben, deren chemische Struktur durch die generische Formel 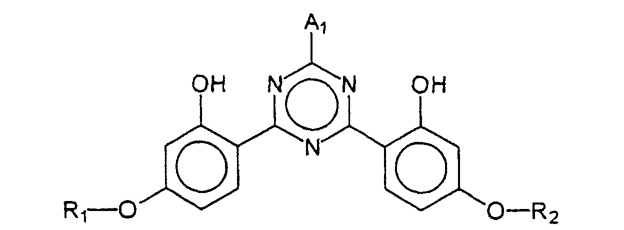 wiedergegeben wird, wobei R₁ , R₂ und A₁ verschiedenste organische Reste repräsentieren.

Vorteilhaft im Sinne der vorliegenden Erfindung sind femer das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 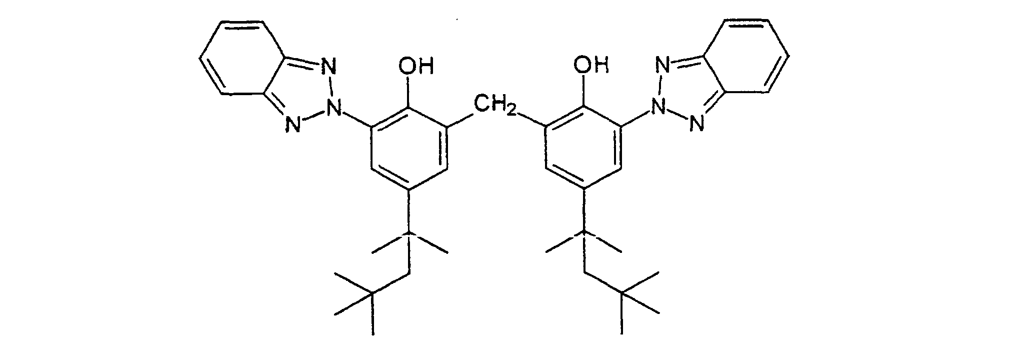 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2Hbenzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 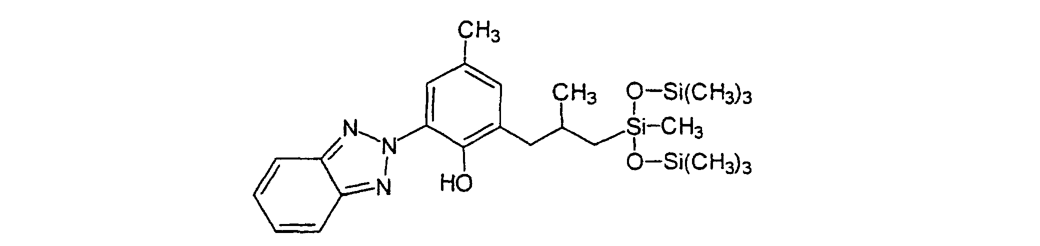 gekennzeichnet ist.

Die UV-B-Filter können öllöslich oder wasserlöslich sein. Vorteilhafte öllösliche UV-B-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester,
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B-Filtersubstanzen sind z. B.:
■ Salze der 2-Phenylbenzimidazol-5-sulfonsäure, wie ihr Natrium-, Kalium- oder ihr Triethanofammonium-Salz, sowie die Sulfonsäure selbst;
■ Sulfonsäure-Derivate des 3-Benzylidencamphers, wie z. B. 4-(2-Oxo-3-bomylidenmethyl)benzolsulfonsäure, 2-Methyl-5-(2-oxo-3-bomylidenmethyl)sulfonsäure und deren Salze.

Eine weitere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

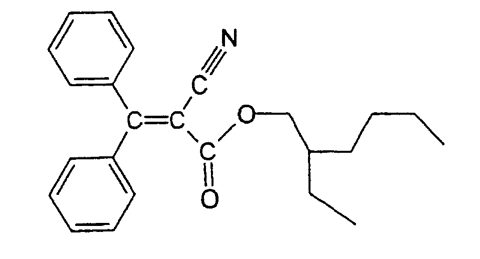

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-Aund/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

| | 1 | 2 | 3 | 4 | 5 | 6 |
|---|---|---|---|---|---|---|
| Glycerylstearatcitrat | 0,5 | 0,5 | | 0,5 | 0,5 | |
| Glycerylstearat | | | 0,5 | | | 0,5 |
| Tributylcitrat | 10 | | 5 | 5 | | 3 |
| Acetyltributylcitrat | | 10 | | 5 | 5 | 3 |
| CapryliGCapric Triglycerid | | 2 | | 2 | | |
| C₁₂₋₁₅ Alkylbenzoat | | 2 | 5 | 2 | 3 | |
| Butylenglykol Dicaprylat/Dicaprat | | 2 | 5 | 2 | | |
| Octyltriazon | | | 0,5 | | | |
| Methylbenzyliden-campher | | | 2 | | | |
| Butylmethoxydibenzoylmethan | | | 2 | | | |
| Titandioxid | | | | 3 | | |
| Vitamin E-Acetat | | | 0,5 | 0,5 | | |
| Konservierung | 0,7 | 0,7 | 0,7 | 0,7 | 0,7 | 0,7 |
| Xanthan Gum | | | | 0,1 | 0,1 | |
| Carbomer | | 0,1 | | | | 0,05 |
| Glycerin | 10 | 1,6 | 10 | 9 | | 10 |
| NaCl | | | | | | 3,8 |
| Natrium-Phenylbenzimidazolsulfonat | 3,5 | | 8 | | | |
| Wasser | 75,3 | 81,1 | 60,8 | 70,2 | 90,7 | 78,95 |

## Patentansprüche

1. Kosmetische oder dermatologische Zubereitungen vom Typ Öl-in-Wasser, welche eine Ölphase, in weiche ein oder mehrere Citronensäureester gewählt aus der Gruppe Acetyltriethylcitrat, Tributylcitrat und Acetyltributylcitrat eingearbeitet sind, und eine Wasserphase enthalten, wobei die Dichtedifferenz zwischen der Ölphase und der Wasserphase, bestimmbar mit einem rechnenden digitalen Dichtemesser vom Typ DMA 45 der Firma chempro/PA bei 25 °C, nicht größer als 0,01 g·cm⁻³ ist.

2. Zubereitung nach Anspruch 1, **dadurch gekennzeichnet, daß** zusätzlich weitere kosmetische oder pharmazeutische Hilfs-, Zusatz- und/oder Wirkstoffe enthalten sind.

3. Zubereitung nach einem der vorhergehenden Ansprüche, **dadurch gekennzeichnet, daß** die Viskosität dieser Zubereitung kleiner als 10.000 mPa·s, bestimmbar mit einem Haake Viskotester VT-02 bei 25 °C, ist.

4. Zubereitung nach einem der vorhergehenden Ansprüche, **dadurch gekennzeichnet, daß** die Viskosität dieser Zubereitung kleiner als 2.000 mPa·s, insbesondere kleiner als 1.500 mPa·s, bestimmbar mit einem Haake Viskotester VT-02 bei 25 °C, ist.

5. Zubereitung nach einem der vorhergehenden Ansprüche, **dadurch gekennzeichnet, daß** die Zubereitung sprühbar ist.

6. Zubereitung nach einem der vorhergehenden Ansprüche, **dadurch gekennzeichnet, daß** der Durchmesser der Öltröpfchen im Durchschnitt kleiner als 50 µm ist.

7. Zubereitung nach einem der vorhergehenden Ansprüche, **dadurch gekennzeichnet, daß** die Dichte der Gesamtformulierung größer als 0,9 g·cm⁻³, insbesondere größer als 0,95 g·cm⁻³ ist.

8. Zubereitung nach einem der vorhergehenden Ansprüche, **dadurch gekennzeichnet, daß** der Gehalt an einem oder mehreren Emulgatoren kleiner ist als 1 Gew.-%. bezogen auf das Gesamtgewicht der Zubereitung.

9. Zubereitung nach einem der vorhergehenden Ansprüche, **dadurch gekennzeichnet, daß** der Gehalt an einem oder mehreren Verdickungsmitteln zwischen 0,05 Gew.-% und 0,15 Gew.-% ist, bezogen aus das Gesamtgewicht der Formulierung.

10. Nicht-therapeutische Verwendung von Zubereitungen nach einem der vorhergehenden Ansprüche als kosmetisches oder dermatologisches Lichtschutzmittel.

11. Verfahren zur Stabilisierung von O/W-Formutierungen, **dadurch gekennzeichnet, daß** die Dichte der Ölphase durch Zugabe von einem oder mehreren Citronensäureestem der allgemeinen Strukturformel 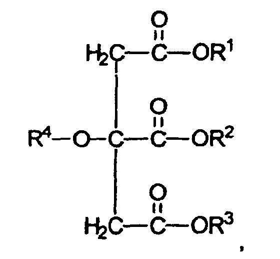 worin R¹, R² und R³ unabhängig voneinander aus der Gruppe Ethyl, Propyl und Butyl gewählt werden und R⁴ ein Wasserstoffatom oder eine CH₃(CO)-Gruppe darstellt, der Dichte der Wasserphase derart angeglichen wird, daß die Dichtedifferenz der beiden Phasen, bestimmbar mit einem rechnenden digitalen Dichtemesser vom Typ DMA 45 der Firma chempro/PA bei 25 °C, nicht größer als 0,01 g·cm⁻³ ist.

## Claims

1. A cosmetic or dermatological preparation of the oil-in-water type which comprises an oil phase into which one or more citric esters selected from the group consisting of acetyltriethyl citrate, tributyl citrate and acetyltributyl citrate are incorporated and a water phase, where the difference in density between the oil phase and the water phase, determinable using a computerized digital density meter of the type DMA 45 from chempro/PA at 25°C, is not greater than 0.01 g·cm⁻³.

2. The preparation as claimed in claim 1, wherein further cosmetic or pharmaceutical auxiliaries, additives and/or active ingredients are additionally present.

3. The preparation as claimed in any of the preceding claims, wherein the viscosity of this preparation is less than 10 000 mPa·s, determinable using a Haake viscometer VT-02 at 25°C.

4. The preparation as claimed in any of the preceding claims, wherein the viscosity of this preparation is less than 2 000 mPa·s, in particular less than 1 500 mPa·s, determinable using a Haake viscometer VT-02 at 25°C.

5. The preparation as claimed in any of the preceding claims, wherein the preparation is sprayable.

6. The preparation as claimed in any of the preceding claims, wherein the diameter of the oil droplets is, on average, less than 50 µm.

7. The preparation as claimed in any of the preceding claims, wherein the density of the overall formulation is greater than 0.9 g·cm⁻³, in particular greater than 0.95 g·m⁻³.

8. The preparation as claimed in any of the preceding claims, wherein the content of one or more emulsifiers is less than 1% by weight, based on the total weight of the preparation.

9. The preparation as claimed in any of the preceding claims, wherein the content of one or more thickeners is between 0.05% by weight and 0.15% by weight, based from [sic] the total weight of the formulation.

10. The nontherapeutic use of the preparation as claimed in any of the preceding claims as cosmetic or dermatological light protection agent.

11. A method of stabilizing O/W formulations, which comprises matching the density of the oil phase to the density of the water phase by adding one or more citric esters of the general structural formula 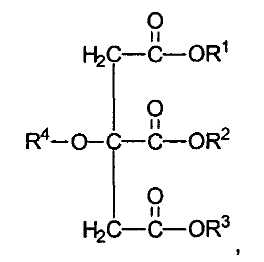 in which R¹, R² and R³, independently of one another, are chosen from the group consisting of ethyl, propyl and butyl, and R⁴ is a hydrogen atom or a CH₃(CO) group, in such a way that the difference in density between the two phases, determinable using a computerized digital density meter of the type DMA 45 from chempro/PA at 25°C, is not greater than 0.01 g·cm⁻³.

## Revendications

1. Préparations cosmétiques ou dermatologiques du type huile-dans-eau, qui contiennent une phase huileuse dans laquelle sont incorporés un ou plusieurs esters d'acide citrique, choisis dans le groupe constitué par le citrate d'acétyltriéthyle, le citrate de tributyle et le citrate d'acétyltributyle, et une phase aqueuse, la différence de densité entre la phase huileuse et la phase aqueuse, pouvant être déterminée à l'aide d'un densitomètre numérique calculateur du type DMA 45 de la société Chempro/PA à 25°C, n'étant pas supérieure à 0,01 g.cm⁻³.

2. Préparation selon la revendication 1, **caractérisée en ce qu'**elle contient en outre d'autres adjuvants, additifs et/ou substances actives cosmétiques ou pharmaceutiques.

3. Préparation selon l'une quelconque des revendications précédentes, **caractérisée en ce que** la viscosité de cette préparation, pouvant être déterminée à l'aide d'un viscosimètre Haake VT-02 à 25°C, est inférieure à 10 000 mPa.s.

4. Préparation selon l'une quelconque des revendications précédentes, **caractérisée en ce que** la viscosité de cette préparation, pouvant être déterminée à l'aide d'un viscosimètre Haake VT-02 à 25°C, est inférieure à 2 000 mPa.s, en particulier inférieure à 1 500 mPa.s.

5. Préparation selon l'une quelconque des revendications précédentes, **caractérisée en ce que** la préparation est pulvérisable.

6. Préparation selon l'une quelconque des revendications précédentes, **caractérisée en ce que** le diamètre des gouttelettes d'huile est en moyenne inférieur à 50 *µ*m.

7. Préparation selon l'une quelconque des revendications précédentes, **caractérisée en ce que** la densité de la composition totale est supérieure à 0,9 g.cm⁻³, en particulier supérieure à 0,95 g.cm⁻³.

8. Préparation selon l'une quelconque des revendications précédentes, **caractérisée en ce que** la teneur en un ou plusieurs émulsifiants est inférieure à 1 % en poids, par rapport au poids total de la préparation.

9. Préparation selon l'une quelconque des revendications précédentes, **caractérisée en ce que** la teneur en un ou plusieurs épaississants est comprise entre 0,05 % en poids et 0,15 % en poids, par rapport au poids total de la composition.

10. Utilisation non thérapeutique de préparations selon l'une quelconque des revendications précédentes, en tant que photoprotecteur cosmétique ou dermatologique.

11. Procédé pour la stabilisation de compositions H/E, **caractérisé en ce que** la densité de la phase huileuse est égalisée à la densité de la phase aqueuse, par addition d'un ou plusieurs esters d'acide citrique de formule générale suivante 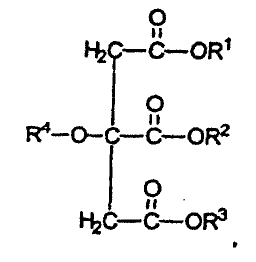 dans laquelle R¹, R² et R³ sont choisis, indépendamment les uns des autres, dans l'ensemble constitué par les groupes éthyle, propyle et butyle, et R⁴ représente un atome d'hydrogène ou un groupe CH₃(CO), de sorte que la différence de densité des deux phases, pouvant être déterminée à l'aide d'un densitomètre numérique calculateur du type DMA 45 de la société Chempro/PA à 25°C, n'est pas supérieure à 0,01 g.cm⁻³.
